# EUROPEAN PATENT SPECIFICATION

(11) **EP 2 526 896 B1**
(45) Date of publication and mention of the grant of the patent: **07.10.2015**
(21) Application number: 12180173.2
(22) Date of filing: 13.04.2006
(51) Int. Cl.: A61F 2/14

(54) **Implantable lenses with modified edge regions**
Implantierbare Linsen mit modifizierten Kantenabschnitten
Lentilles implantables présentant des bords modifiés

(30) Priority: 15.04.2005 US 106983
(43) Date of publication of application: 28.11.2012
(62) Divisional of application: 06750406.8
(73) Proprietor: ReVision Optics, Inc., Lake Forest, CA 92630 (US)
(72) Inventor: Miller, Troy A., Rancho Santa Margarita, CA California 92688 (US); Cunanan, Crystal M., Mission Viejo, CA California 92691 (US); Vatz, Alexander, Lake Forest, CA California 92630 (US)
(74) Representative: Price, Nigel John King

(56) References cited:
- US-A- 5 123 921
- US-A1- 2005 080 484
- US-B2- 6 875 232

## Description

### FIELD OF THE INVENTION

The field of the invention relates generally to implantable lenses and, more particularly, to implantable lenses having modified edge regions.

### BACKGROUND INFORMATION

As is well known, abnormalities in the human eye can lead to vision impairment. Some typical abnormalities include variations in the shape of the eye, which can lead to myopia (near-sightedness), hyperopia (far-sightedness) and astigmatism as well as variations in the tissue present throughout the eye, such as a reduction in the elasticity of the lens, which can lead to presbyopia. Certain devices, generally referred to as implantable lenses, have been used to successfully treat these and other types of vision impairment.

Implantable lenses typically fall into one of two categories: intraocular lenses (IOLs), which may be implanted deep within the eye to replace the eye's natural crystalline lens, and corneal implants, which are typically implanted near the surface of the eye in the cornea to alter the incident light. Corneal implants, in turn, can be classified as an onlay or an inlay. An onlay is an implant that is placed over the cornea such that the outer layer of the cornea, e.g., the epithelium, can grow over and encompass the implant. An inlay is an implant that is surgically implanted into the cornea beneath a portion of the corneal tissue using, for instance, keratophakia. Example methods of implanting a corneal inlay are described in further detail in US 2005/017 8394 A1 filed August 23, 2004, and entitled "Method for Keratophakia Surgery."

Because corneal implants are placed within the corneal tissue, a significant concern lies in preventing the tissue from adversely reacting to the implant and creating undesirable conditions. For instance, certain adverse tissue reactions, such as cellular secretions and keratocyte build-up, can lead to an undesirable condition referred to as corneal haze. Corneal haze can obstruct the passage of light through the cornea and the implant and thus prevent proper treatment of the visual impairment. Although corneal haze is multifactorial, there is evidence that it can be influenced, at least in part, by mechanical forces placed on the keratocytes in the corneal tissue.

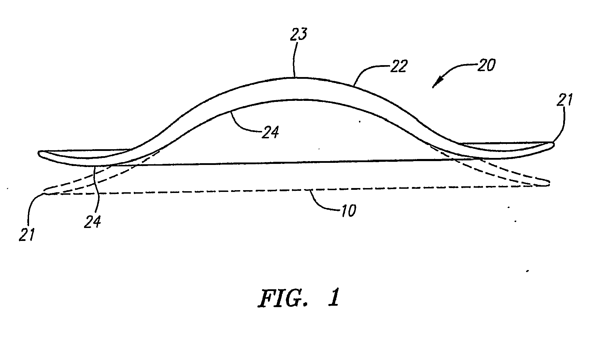

Furthermore, some corneal implants that are relatively flat around the outer edges, such as aspherical implants and shallow spherical implants to name a few, can suffer from edge lift. Edge lift occurs when the anterior surface of the implant around the outer edge tends to curve or lift back towards the apex. FIG. 1 is a cross-sectional view of a conventional corneal implant 20 suffering from edge lift, which is exaggerated for the purposes of illustration. Here, the implant 20 has an outer edge 21, an anterior surface 22, an apex 23 and a posterior surface 24. An ideal edge profile is indicated by dashed line 10. In the ideal case, the most posterior point on the anterior surface 22 is located at the outer edge 21. However, in a lens suffering from edge lift the most posterior point of the anterior surface 22 can be located at a position 24 closer to the apex 23 than the outer edge 21. Edge lift can progress and build up with time post-genetively and result in deteriorated optical performance and can also make the implantation procedure more difficult.

Accordingly, there is a need for improved implantable lenses that reduce adverse physiological reactions to the presence of the lens and decrease the risk of edge lift.

US 6 875 232 B2 discloses a corneal implant formed of a biocompatible, permeable, micro-porous hydrogel with a refractive index in the range of 1.36-1.39 so as to be substantially similar to the refractive index of the cornea. The implant is generally circular in shape and is of a size greater than the size of the pupil in normal or bright light and can specifically be used to correct presbyopia. For simple or compound presbyopia, the implant is made by modifying the radius of curvature in the central 1.5-3mm, thereby forming a multi-focal outer corneal surface where the central portion of the cornea achieves an added plus power for close-up work.

US 5 123 921 A discloses a lens for implantation into the cornea of the eye for providing correction of myopia. The lens of is a one-piece member having an anterior surface, a posterior surface and a peripheral transition zone which serves to join the anterior and posterior surfaces. The overall configuration of the lens is circular as viewed from the central axis of the lens. The anterior surface of the lens is convex and the posterior surface is concave. The anterior and posterior surfaces are joined at the periphery of the lens by a peripheral transition zone. The peripheral transition zone is formed of a first annular surface, a second annular surface and a third annular surface. The first annular surface is in a plane perpendicular to the central axis of the lens and abuts the posterior surface. The second annular surface is also generated about a point along the central axis, is perpendicular to and abuts the first annular surface and is in a plane parallel to the line of the central axis. The third annular surface is an annular spherical segment and abuts both the second annular surface and the anterior surface.

### SUMMARY

According to the present invention there is provided the corneal inlay of claim 1.

Additional aspects of the corneal inlay are set out in the dependent claims.

Also disclosed hereinafter are implantable lenses having a body with a first region and a second region, the first region having a first refractive index and a second region having a second refractive index different from the first refractive index. The first region can be permeable to an amount of fluid and nutrients sufficient to substantially sustain tissue adjacent to the body. The second region can have the same permeability as the first region or it can be relatively less permeable than the first region. The first and second regions can provide refractive correction over any distances desired (i.e., near/far, far/near etc.) and can be arranged in any desired manner. The lens can have an anterior surface with any curvature desired and can be configured as a corneal inlay or onlay. In another example, the first region can be composed of a first polymeric material and the second region can be composed of a second polymeric material, where the first and second regions are integrally coupled together. Any number of regions two or greater can be included as desired with one or more regions integrally coupled together.

Also disclosed is an exemplary method of manufacturing an implantable lens, where the method includes forming a first core comprising a first polymer having a first refractive index, forming an interface region around at least a portion of the first core, forming a second core comprising a second polymer around at least a portion of the interface region, the second polymer having a second refractive index different than the first refractive index and forming an implantable lens from the first and second cores. The interface region can include a mixture of the first and second polymers and can have a third refractive index different from the first and second refractive indices and can be used to provide additional refractive correction or to serve as a gradual transition between the first and second polymeric regions. The interface region can integrally couple the first and second cores together and can include an interpenetrating network of the first polymer and second polymer.

The example method can also include placing a monomeric solution in contact with the first core, where the first polymer is soluble in the monomeric solution, dissolving a portion of the first core in the monomeric solution such that the monomeric solution and the dissolved portion of the first core mix in the interface region, and polymerizing the mixture of the monomeric solution and the dissolved portion of the first core in the interface region.

There is also hereinafter disclosed an implantable lens having a body including a first substantially aspherical surface having a first asphericity (Q) and a second substantially aspherical surface having a second asphericity (Q) different from the first asphericity. The first and second aspherical surfaces can be configured to assist vision at any desired distance or range of distances from the eye and can be arranged in any fashion desired.

Other systems, methods, features and advantages of the invention will be or will become apparent to one with skill in the art upon examination of the following figures and detailed description. It is intended that all such additional systems, methods, features and advantages be included within this description, be within the scope of the invention, and be protected by the accompanying claims. It is also intended that the invention not be limited to the details of the example embodiments.

### BRIEF DESCRIPTION OF THE FIGURES

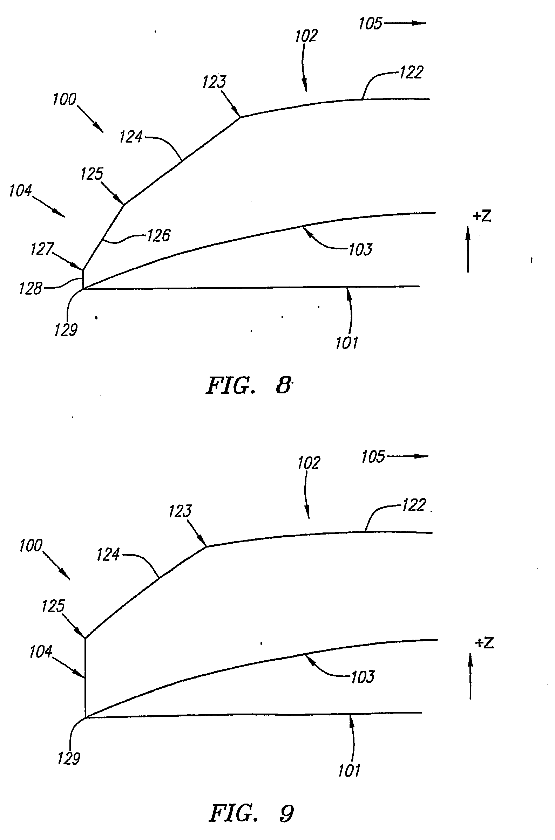

The details of the invention, including fabrication, structure and operation, may be gleaned in part by study of the accompanying figures, in which like reference numerals refer to like segments. Of the illustrated lenses, only the lens of Fig. 9 is an embodiment in accordance with the present invention.
FIG. 1 is a cross-sectional view of a conventional implantable lens.
FIG. 2A is a perspective view depicting an example of an implantable lens.
FIG. 2B is a top-down view depicting another example of the implantable lens.
FIGs. 2C-E are cross-sectional views taken along line 1-1 of FIG. 2B depicting additional examples of the implantable lens.
FIG. 3 is a cross-sectional view depicting an anterior portion of a human eye with an example of the lens implanted therein.
FIGs. 4-8 are cross-sectional views taken along line 1-1 of FIG. 2B depicting additional examples of the implantable lens.
FIG. 9 is a cross-sectional view taken along line 1-1 of FIG. 2B depicting an example embodiment of the implantable lens.
FIG. 10A is a top-down view depicting another example of the implantable lens.
FIG. 10B is a cross-sectional view taken along line 2-2 of FIG. 10A depicting another example of the implantable lens.
FIG. 11A is a perspective view depicting another example of the implantable lens.
FIG. 11B is a top-down view depicting another example of the implantable lens.
FIGs. 11C-D are cross-sectional views taken along line 3-3 of FIG. 11B depicting additional examples of the implantable lens.
FIGs. 12A-D are block diagrams depicting an example method of manufacturing the implantable lens.
FIG. 13 is a cross-sectional view depicting another example of the implantable lens.
FIG. 14A is a top-down view depicting another example of the implantable lens.
FIGs. 14B-C are cross-sectional views taken along line 4-4 of FIG. 14A depicting additional examples of the implantable lens.

### DETAILED DESCRIPTION

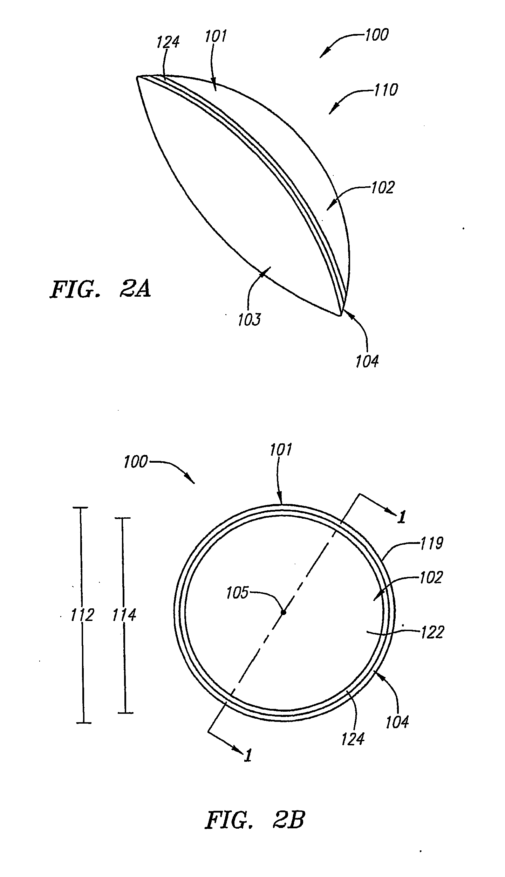

Described herein are improved implantable lenses with modified edge regions that can reduce stimulation of adverse tissue reactions in proximity to the lens. FIGs. 2A-E depict various views of an example of implantable lens 100. FIG. 2A is a perspective view depicting implantable lens 100, where lens 100 has lens body 101, anterior surface 102, posterior surface 103 and outer edge surface 104. FIG. 2B is a top-down view of lens 100 taken in direction 110. Here it can be seen that lens body 101 has a generally circular outer profile 119 with central apex 105 representing the most anterior point of anterior surface 102. Diameter 112 represents the overall diameter of lens body 101 and diameter 114 represents the diameter of corrective portion 122, which is the portion of anterior surface 102 configured to provide correction for one or more specific visual impairments.

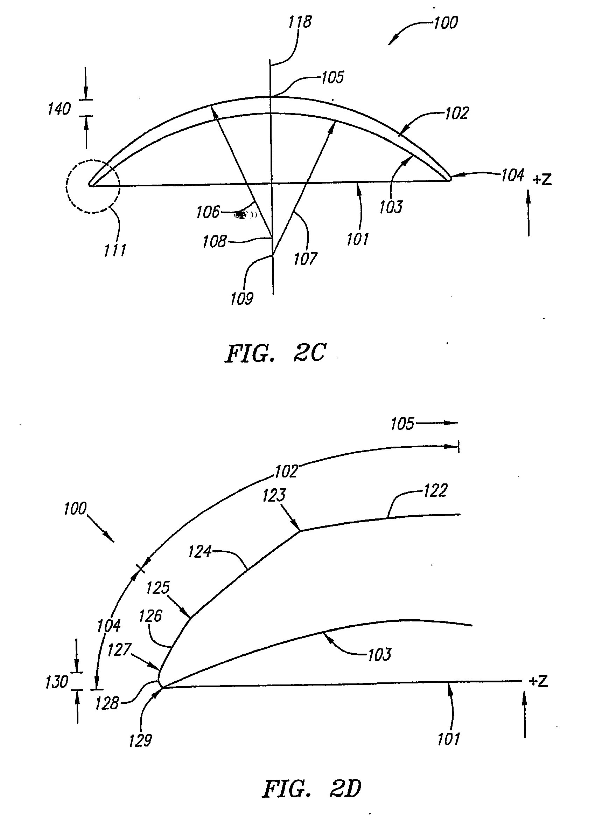

FIG. 2C is a cross-sectional view of lens 100 taken along line 1-1 of FIG. 2B. From this view it can be seen that anterior surface 102 is substantially spherical with radius of curvature 106 measured from vertex 108 located on central axis 118, which intersects apex 105. Likewise, posterior surface 103 also has its own radius of curvature 107 measured from vertex 109. The corrective power of lens 100 is dependent upon these radii 106-107 and can be varied as desired by adjustment of either radii 106-107. It can also be seen here that lens 100 is configured to correct for hyperopia, i.e., the relation of anterior surface 102 to posterior surface 103 gives lens body 101a converging meniscus-like shape along line 1-1. The thickness of lens body 101 along central axis 118 is referenced as center thickness 140.

FIG. 2D is an enlarged cross-sectional view of lens 100, showing region 111 of FIG. 2C in greater detail. In FIG. 2D, corrective portion 122 of anterior surface 102 is substantially spherical and anterior surface 102 also includes a beveled portion 124. Here, beveled portion 124 is curved with a single radius of curvature and is referred to as bevel radius 124. As used herein, "bevel" is defined to include flat surfaces, curved surfaces and surfaces of any other shape. Bevel radius 124 abuts spherical portion 122 at interface 123. Adjacent to bevel radius 124 is outer edge surface 104, the abutment between bevel radius 124 and outer edge surface 104 being referenced as interface 125. Outer edge surface 104 includes first portion 126 and second portion 128, which abut each other at interface 127. Second edge surface portion 128 abuts posterior surface 103 at interface 129. Here, first edge surface portion 126 is curved and is referred to as edge radius 126. In this lens, edge thickness 130 is defined as the height of second edge surface portion 128 in the Z direction from the most posterior point of lens body 101 (interface 129 in this instance) to interface 127.

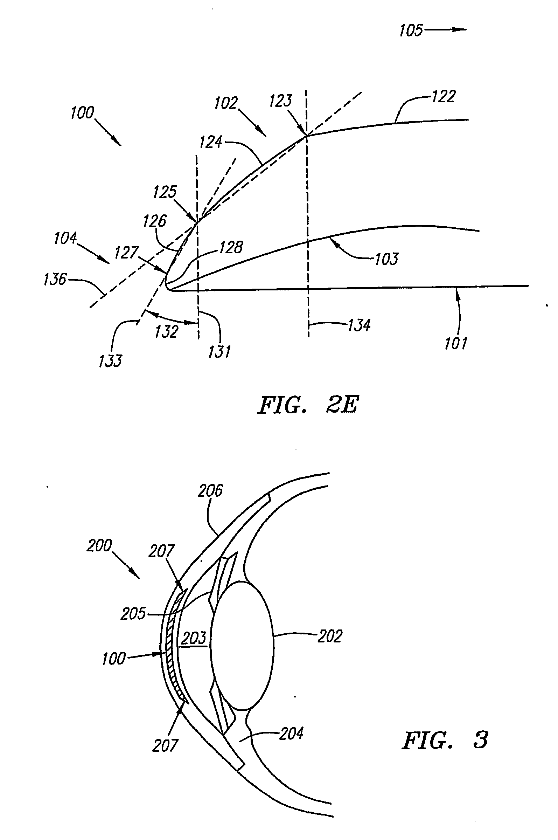

FIG. 2E is another cross-sectional view of region 111 depicting the example of FIG. 2D with edge radius slope angle 132, which defines the slope of edge radius 126. Edge radius slope angle 132 can be defined as the angle between axes 131 and 133. Here, axis 131 is parallel to central axis 118 and intersects interface 125, while axis 133 intersects interfaces 125 and 127. Also depicted here is bevel radius slope angle 135, which defines the slope of bevel radius 124. Bevel radius slope angle 135 can be defined as the angle between axes 134 and 136. Here, axis 134 is parallel to central axis 118 and intersects interface 123 and axis 136 intersects interfaces 123 and 125.

As can be seen in FIGs. 2D-E, edge radius 126 preferably slopes in the -Z direction to a greater degree than bevel radius 124, so that edge radius 126 converges towards posterior surface 103 at a greater rate than bevel radius 124. Stated in terms of slope angles, edge radius slope angle 132 is preferably smaller than bevel radius slope angle 135. As a result, lens 100 is less susceptible to edge lift. Also, the gradual transition between spherical portion 122 and posterior surface 103 can reduce stimulation of adverse tissue reactions to lens 100.

For instance, FIG. 3 is a cross-sectional view depicting an anterior portion of human eye 200 including lens 202, aqueous humor 203, ciliary body 204, iris 205 and cornea 206 with an example of lens 100 implanted therein. Here, lens 100 is shown implanted as a corneal inlay although, it should be noted that lens 100 can also be implanted as a corneal onlay in a position closer to the anterior surface of cornea 206. The gradual transition in the edge region of lens 100 facilitates the acceptance of lens 100 by the surrounding corneal tissue 207, more so than conventional lenses with an unbeveled sharp or steep transition between the anterior and posterior surfaces. As a result, lens 100 is less susceptible to undesirable conditions such as corneal haze and the like. In addition, during the implantation procedure, the modified edge region of lens 100 makes it easier to ascertain whether lens 100 is properly oriented or whether lens 100 is inverted.

In order to sustain the cornea 206 and prevent tissue necrosis, an adequate level of fluid and nutrient transfer should be maintained within cornea 206. Accordingly, lens body 101 is preferably composed of a material with a permeability sufficient to allow fluid and nutrient transfer between corneal tissue 207 adjacent to anterior surface 102 and posterior surface 103, in order to sustain the cornea over a desired period of time. For instance, in one example lens body 101 is composed of a microporous hydrogel material. Microporous hydrogels are described in further detail in U.S. Patent No. 6,875,232 entitled "Corneal Implant and Method of Manufacture."

TABLE 1 depicts example values for one embodiment of a 5.0 millimeter (mm) diameter lens 100 having a given diopter. These example values are for purposes of illustration only and in no way limit the implantable lens 100 to only these or similar values.

**TABLE 1**

| | |
|---|---|
| Diopter | +2.25 |
| Lens diameter 112 (mm) | 5.00 |
| Corrective diameter 114 (mm) | 4.90 |
| Posterior radius 107 (mm) | 7.50 |
| Center thickness 140 (mm) | 0.030 |
| Bevel radius 124 (mm) | 5.500 |
| Edge radius 126 (mm) | 0.025 |
| Edge thickness 130 (mm) | 0.010 |
| Edge slope angle 132 (degrees) | 50 |

The values of edge thickness 130, edge radius 126, edge slope angle 132 and bevel radius 124 are interdependent and based on the desired corrective values, the overall lens diameter 112, the diameter of corrective portion 122, and the shape of anterior surface 102 and posterior surface 103. Preferably, a lens diameter 112 in the range of about 1-10 mm with a corrective portion diameter 114 of about 0.5 mm or greater will have an edge thickness less than or equal to about 0.015 mm, an edge radius 126 in the range of about 0.001-1 mm, an edge slope angle 132 between 0 and 90 degrees and a bevel radius 124 in the range of about 1-10 mm. These ranges are for illustrative purposes only and in no way limit the embodiments described herein.

It should be noted that the modified edge described herein can be used with any type, shape or configuration of implantable lens. For instance, lens 100 can be either a corneal inlay or onlay. Lens 100 can be configured to treat any visual impairment including, but not limited to, myopia, hyperopia, astigmatism, and presbyopia. Lens 100 can also be configured to treat any combination of visual impairments including, but not limited to, presbyopia with myopia or hyperopia and presbyopia with astigmatism. The overall outer profile 119 of lens 100 can be any shape, including, but not limited to, circular, elliptical, irregular, multi-sided, and shapes having an inner aperture. Outer edge surface 104 can configured with outcroppings such as fixation elements and the like. Also, lens body 101 can be fabricated from one or more different materials having any desired refractive index. Furthermore, as will be described in greater detail below, corrective portion 122 of anterior surface 102 can be substantially spherical with or without multiple focal zones, substantially aspherical with or without multiple aspherical surfaces, or any combination and the like. As used herein, the term substantially is intended to broaden the modified term. For instance, a substantially spherical surface does not have to be perfectly spherical, but can include non-spherical variations or errors and the like to a degree sufficient for implementation.

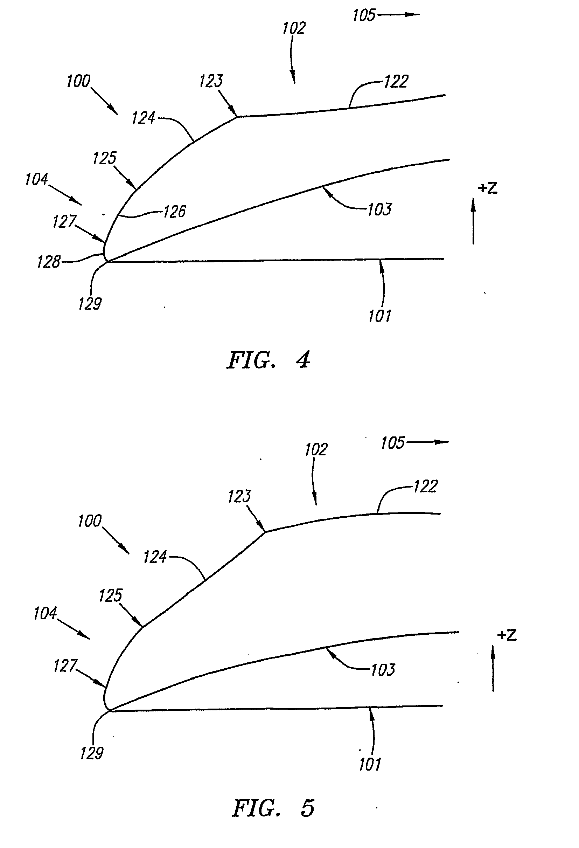

FIGs. 4-9 are cross-sectional views depicting additional examples of lens 100 taken along line 1-1 in region 111 of FIG. 1B. In the example depicted in FIG. 4, corrective portion 122 of anterior surface 102 is substantially aspherical. The rate of curvature of aspherical surfaces typically decreases or increases as the surface progresses outwards towards outer edge surface 104. In this example, the rate of curvature of aspheric surface 122 decreases such that the surface is flatter near outer edge surface 104 than near apex 105 (not shown). Anterior surface 102 and posterior surface 103 diverge as the surfaces 102-103 progress radially outwards from apex 105 (not shown) towards interface 123. From interface 123 to interface 125, bevel radius 124 preferably converges towards posterior surface 103. Likewise, from interface 125 to interface 127, edge radius 126 also preferably converges towards posterior surface 103.

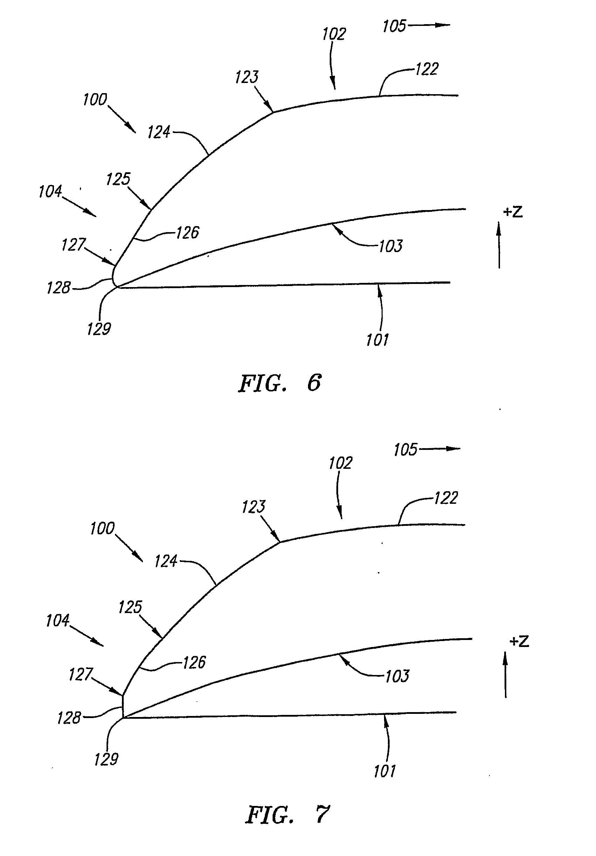

Beveled portion 124 of anterior surface 102 can be flat or curved or any other desired shape. For instance, in FIGs. 2C-E, beveled portion 124 is spherically curved, however, it should be noted that any type of curve can be used. In the example depicted in FIG. 5, beveled portion 124 is flat. Likewise, first and second edge surface portions 126 and 128 can be flat or curved or any other desired shape. For instance, in FIGs. 2C-E, edge radius 126 is substantially spherically curved and second edge surface portion 128 is curved at a variable rate. In the example depicted in FIG. 6, first edge surface portion 126 is flat, while in the example of FIG. 7 second edge surface portion 128 is flat. Any combination of flat and curved surfaces can be implemented. For instance, in FIG. 8, beveled portion 124, and first and second edge surface portions 126 and 128 are all flat. Also, edge surface 104 can be implemented in any desired manner. For instance, in the embodiment of FIG. 9, edge surface 104 is flat and oriented in only the Z direction.

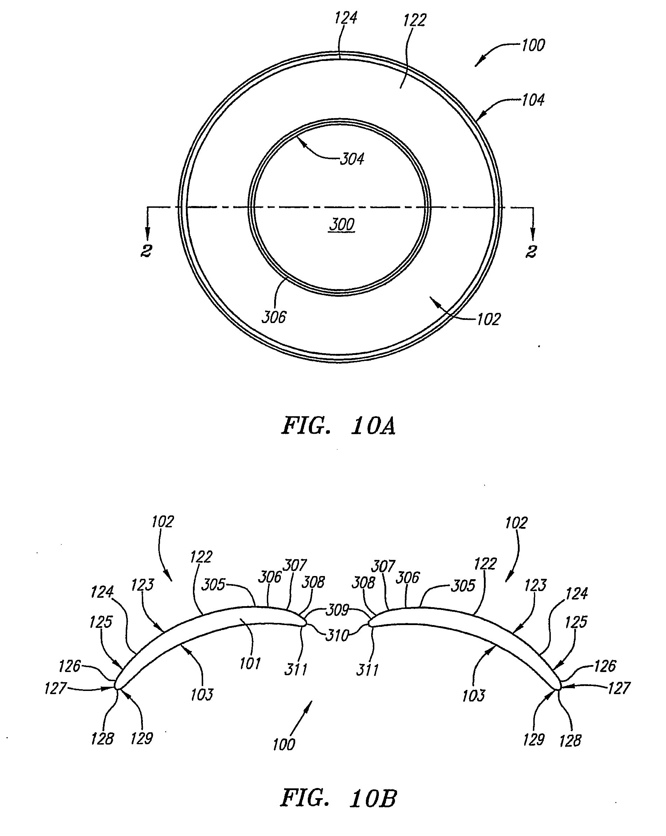

FIG. 10A is a top-down view depicting another example of lens 100 having a ring-like shape. Here, lens 100 includes inner aperture 302 and inner edge surface 304. FIG. 10B is a cross-sectional view of the lens 100 depicted in FIG. 10A taken along line 2-2. Here, it can be seen that anterior surface 102 also includes inner beveled portion 306 located between corrective portion 122 and inner edge surface 304. Like outer edge surface 104, inner edge surface 304 includes first portion 308 and second portion 310, which, in this example, are both curved. Beveled portion 306 abuts corrective portion 122 at interface 305 and first portion 308 abuts beveled portion 306 at interface 307. Second portion 310 abuts first portion 308 at interface 309 and abuts posterior surface 103 at interface 311. It should be noted that edge surface 304 and beveled portion 306, like edge surface 104 and beveled portion 124 described above, can be shaped or configured in any manner desired. Lenses 100 of the type depicted in FIGs. 10A-B are described in more detail in US 2005/0119738 A1, entitled "Myopic Corneal Ring with Central Accommodating Portion" and filed January 11, 2005.

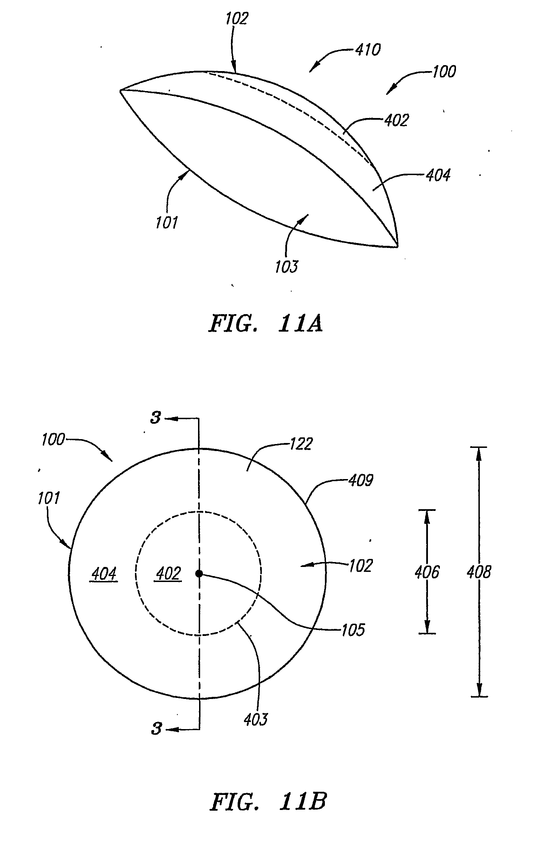

As mentioned above, lens 100 with the modified edge region as described herein can also be implemented as a multifocal lens. FIG. 11A is a perspective view depicting an example of implantable lens 100 configured to provide multifocal correction. Here, lens 100 includes two corrective regions 402 and 404 each having a different refractive index. The different refractive indices in each region allow for correction of visual impairments over different distance ranges. For instance, the refractive indices of regions 402 and 404 can be predetermined such that region 402 provides refractive correction over relatively near distances while region 404 provides correction over relatively far distances or vice-versa. Any combination and number of two or more corrective regions can be used. Likewise, any refractive index can be used including refractive indices that are substantially similar to cornea 206 (about 1.36 - 1.39) and refractive indices that are greater than or less than that of cornea 206.

FIG. 11B is a top down view depicting this example of lens 100 taken along direction 410. In this example, lens 100 has apex 105, a generally circular outer edge profile 409 and regions 402 and 404 have diameters 406 and 408, respectively. The transition between regions 402 and 404 is referenced as interface 403. Here, regions 402 and 403 are arranged as generally concentric circular regions. It should be noted that regions 402 and 403 can be arranged in any desired manner such as eccentric, hemispherical, irregular and the like. Also, any number of two or more regions can be implemented with any number or none of those regions being integrally coupled together.

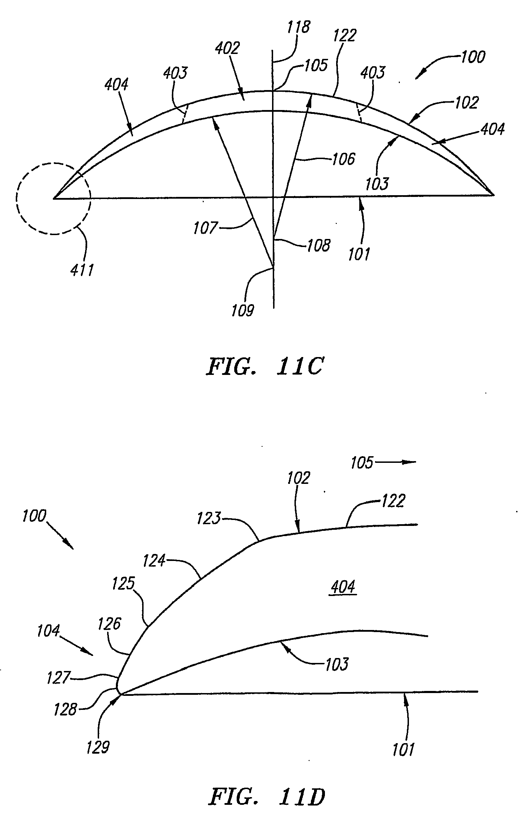

FIG. 11C is a cross-sectional view depicting the example of FIG. 11B taken over line 3-3. Here, corrective portion 122 of anterior surface 102 is substantially spherical having one radius of curvature 106 and posterior surface 103 is also substantially spherical having one radius of curvature 107. Adjustment of these radii 106-107 along with the selection of the appropriate refractive index for regions 402-404 can provide the proper diopter values for each zone to treat a given individual. FIG. 11D is an enlarged cross-sectional view of this example lens 100, showing region 411 of FIG. 11C in greater detail. In this example, similar to the example depicted in FIG. 2D, lens 100 includes bevel radius 124, edge radius 126 and curved second edge surface portion 128.

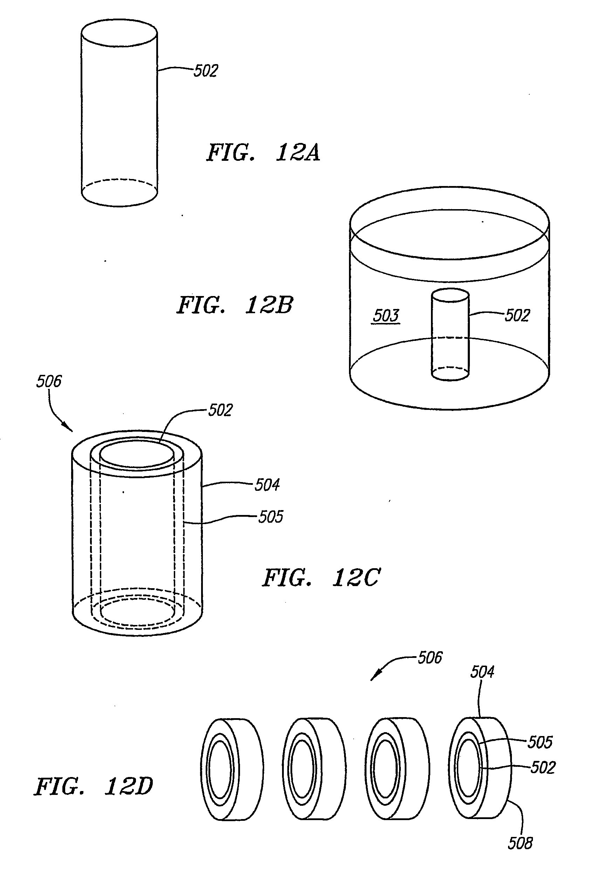

To provide different refractive indices, in one example regions 402 and 404 are fabricated from different materials integrally coupled together at interface 403. For instance, each region 402 and 404 can be fabricated from different microporous hydrogel materials. In one example, lens 100 is fabricated by first forming a solid polymeric cylindrical core 502, such as that depicted in FIG. 12A, which corresponds to region 402 and has approximately the same diameter as diameter 406 of region 402. This core can then be surrounded by a monomeric solution 503 in a manner similar to that depicted in FIG. 12B. Polymeric core 502 is preferably at least slightly soluble in monomeric solution 503. Monomeric solution 503 can then be polymerized to form outer polymeric cylindrical region 504 surrounding inner core 502 as depicted in FIG. 12C. Outer region 504 preferably corresponds to region 404 and has approximately the same diameter or a slightly larger diameter than diameter 408 of region 404. Inner core 502 and outer region 504 together form lens core 506, from which one or more lens can be fabricated, such as, for instance, by separating core 506 into disc-shaped buttons 508 as depicted in FIG. 12D. Each individual button can be machined or cut into the desired shape and further processed (e.g., softened, hydrated, etc.) to form an individual lens body 101.

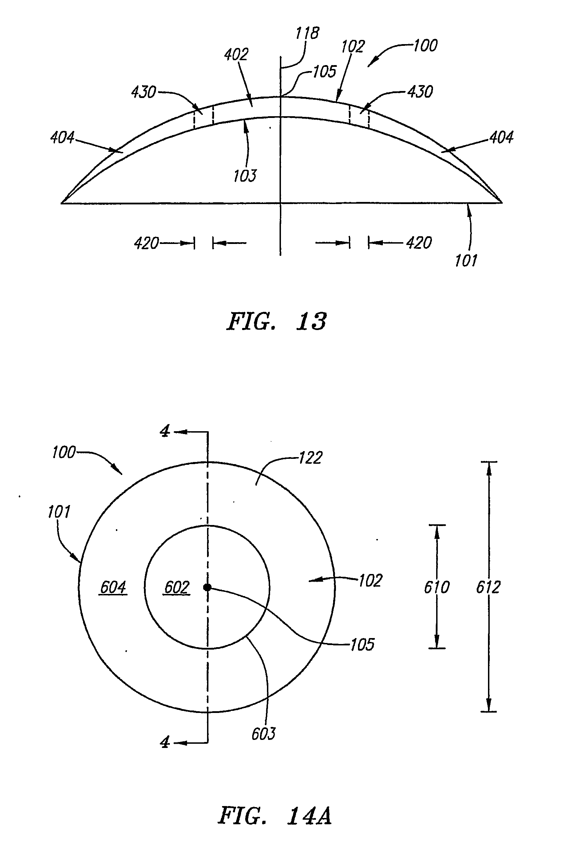

As mentioned above, polymeric core 502 is preferably at least slightly soluble in monomeric solution 503. This is so that solution 503 can dissolve the outer surface of core 502 and become interdispersed and mixed with the dissolved portion of core 502. Once solution 503 is polymerized and solidified, an interface region 505 between cores 502 and 504 can be formed where the different polymers in cores 502 and 504 together form an interpenetrating network. This interface region corresponds to interface region 430 in FIG. 13 below and integrally couples regions 402 and 404 together.

FIG. 13 is a cross-sectional view of an example of lens 100 having interface region 430. By integrally coupling regions 402 and 404 together, interface region significantly reduces the risk that regions 402 and 404 will separate, such as can be the case when an adhesive is used to join regions 402 and 404. Furthermore, interface region 430 can have a refractive index or range of refractive indices between the refractive indices of regions 402 and 404. As a result, interface region 430 can act as an optical transition between regions 402 and 404 and add a third multifocal region to lens 100. This can eliminate an immediate or sharp transition between the refractive indices of regions 402 and 404 that could result in visual artifacts such as halo or glare.

The width 420 of interface region 430 can be varied as desired. For instance, to generate a wider interface region 430, monomeric solution 504 can be left in contact with inner core 502 for a longer period of time before polymerization, or, the solubility of inner polymeric core 502 in monomeric solution 504 can be increased. Generally, the wider interface region 430 becomes, the more noticeable region 430 to the subject as a multifocal region.

It should be noted that lens 100 can be fabricated in any manner and is not limited to the example described with respect to FIGs. 12A-D. Other polymerization methods known in the art including, but not limited to, dip coating, spinning, casting, and the polymerization of prepolymers, can be used in the formation of regions 402 and 404.

In another example, each region 402 and 404 is configured with varying levels of permeability. For instance, region 402 can have a level of permeability to fluid and nutrients that is sufficient to substantially sustain cornea 206, while region 404 can have a permeability to either fluid or fluid and nutrients that is relatively less than region 402, including being entirely impermeable to fluid and nutrients. This allows for the use of more types of materials having a wider range of refractive indices and/or structural characteristics.

In order to allow enough fluid/nutrient transfer to sustain cornea 206, the size of any impermeable region is preferably minimized. For instance, any circular central region, similar to the example of region 402 described with respect to FIG. 11B, that is impermeable to fluid and nutrients is preferably less than about 3 mm in diameter (diameter 406) or about 7.1 square mm. However, it should be noted that lens 100 is not limited to any one total impermeable surface area, the size and surface area of any impermeable region being dependent on the shape of the region and the relative level of permeability of any accompanying regions. For instance, an example of lens 100 having many concentric regions arranged in a bullseye fashion where the regions alternate between permeable and impermeable could allow for a total surface area of impermeable regions that is greater than 7.1 square mm.

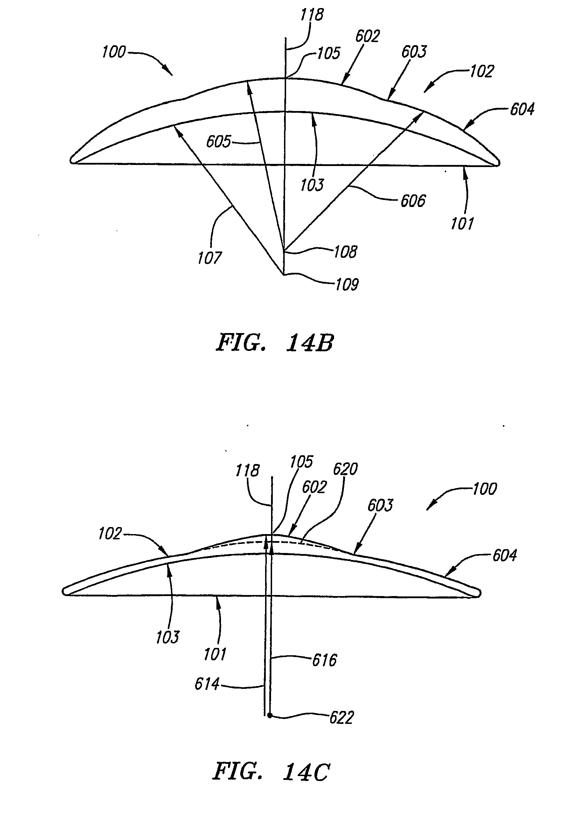

FIG. 14A is a top-down view depicting another example of multifocal lens 100 where corrective portion 122 of anterior surface 102 includes surfaces 602 and 604 having different rates of curvature. Surfaces 602 and 604 have diameters 610 and 612, respectively. FIG. 14B is a cross-sectional view of another example of lens 100 taken along line 4-4 of FIG. 14A. Here, surfaces 602 and 604 are each substantially spherical but have different radii of curvature 605 and 606, respectively. The abutment between surface 602 and 604 is referenced as interface 603. Each surface 602 and 604 can be configured with a different diopter value to correct for separate distances ranges (e.g., near-far, far-near, etc.). TABLE 2 depicts example values for three examples of a 5.0 millimeter (mm) diameter lens 100 having multiple spherical surfaces 602 and 604 similar to that depicted in FIG. 14B. Each of the three examples provides for a different degree of correction for relatively far distances (sphere) and relatively near distances (add). These corrective values are shown in the format "sphere diopter/add diopter." All of these example values are for purposes of illustration only and in no way limit the implantable lens 100 to only these or similar values.

**TABLE 2**

| | | | |
|---|---|---|---|
| Parameter | 0.00/1.75 | 0.00/2.00 | 0.00/2.25 |
| Lens diameter 112 (mm) | 5.00 | 5.00 | 5.00 |
| Posterior radius 107 (mm) | 7.50 | 7.50 | 7.50 |
| Center thickness 140 (mm) | 0.020 | 0.021 | 0.022 |
| Bevel radius 124 (mm) | 4.770 | 4.770 | 4.770 |
| Edge radius 126 (mm) | 0.025 | 0.050 | 0.050 |
| Edge thickness 130 (mm) | 0.010 | 0.010 | 0.010 |
| Edge slope angle 132 (degrees) | 45 | 45 | 45 |
| Spherical Surface 602 | | | |
| Diameter 610 (mm) | 2.00 | 2.00 | 2.00 |
| Radius 605 (mm) | 7.252 | 7.217 | 7.182 |
| Spherical Surface 604 | | | |
| Diameter 612 (mm) | 4.90 | 4.90 | 4.90 |
| Radius 606 (mm) | 7.505 | 7.505 | 7.505 |

FIG. 14C is a cross-sectional view of another example of lens 100 taken along line 4-4 of FIG. 14A. Here, surfaces 602 and 604 are each substantially aspherical. Surfaces 602 and 604 each have a radius 614 and 616, respectively, measured along central axis 118. Radius 616 is measured along central axis 118 from vertex 622 to an imaginary position of surface 604 corresponding to the point where surface 604 would intersect central axis 118 if surface 604 were to extend all the way to central axis 118 as indicated by dashed line 620.

Because aspherical surfaces are inherently multifocal, the inclusion of multiple aspherical surfaces provides an added dimension of multifocality to lens 100. For instance, surface 602 can have any asphericity (Q) and can provide a range of diopter values varying at any rate from apex 105 to interface 603 and can be configured to provide for correction over relatively near distances, while surface 604 can have a range of diopter values varying at any rate from interface 603 to interface 123 and can be configured to provide correction over relatively far distances. One of skill in the art will readily recognize that each surface 602 and 604 can have any range of diopter values and provide for correction over any distance.

TABLE 3 depicts example values for one example of a 5.0 millimeter (mm) diameter lens 100 having multiple aspherical surfaces 602 and 604 similar to that depicted in FIG. 14C. Each of the three examples provides for a different degree of correction for relatively far distances and relatively near distances. All of these example values are for purposes of illustration only and in no way limit the implantable lens 100 to only these or similar values.

**TABLE 3**

| | | | |
|---|---|---|---|
| Parameter | 0.00/1.75 D | 0.00/2.00 D | 0.00/2.25 D |
| Lens diameter 112 (mm) | 5.00 | 5.00 | 5.00 |
| Posterior radius 107 (mm) | 7.50 | 7.50 | 7.50 |
| Center thickness 140 (mm) | 0.020 | 0.021 | 0.022 |
| Bevel radius 124 (mm) | 4.770 | 4.770 | 4.770 |
| Edge radius 126 (mm) | 0.025 | 0.025 | 0.025 |
| Edge thickness 130 (mm) | 0.010 | 0.010 | 0.010 |
| Edge slope angle 132 (degrees) | 45 | 45 | 45 |
| Aspherical Surface 602 | | | |
| Diameter 610 (mm) | 2.00 | 2.00 | 2.00 |
| Radius 614 (mm) | 7.217 | 7.182 | 7.148 |
| Asphericity (Q) | -1.015 | -1.001 | -0.987 |
| Aspherical Surface 604 | | | |
| Diameter 612 (mm) | 4.90 | 4.90 | 4.90 |
| Radius 616 (mm) | 7.452 | 7.452 | 7.452 |
| Asphericity (Q) | -0.225 | -0.225 | -0.225 |

Although not depicted in FIGs. 14A-C, lens 100 can have one or more transition surfaces at interface 603 that provide for a smoother transition between surfaces 602 and 604, as sharp transitions can stimulate adverse tissue reactions. Edge surface 104 and beveled portion 124 are also not depicted in FIGs. 14A-C, but it can be included as desired. Also, it should be noted that lens 100 can have any number of multifocal surfaces or refractive regions as desired. The multifocal surfaces 602 and 604, substantially spherical or substantially aspherical, can also be arranged in any manner desired including, but not limited to, eccentric, hemispherical, irregular and the like.

In the foregoing specification, the invention has been described with reference to specific embodiments thereof. It will, however, be evident that various modifications and changes may be made thereto without departing from the scope of the claims.

## Claims

1. A corneal inlay for treating presbyopia, comprising:
a diameter (114) from imam to 5mm;
a refractive index between about 1.36 and 1.39; and
an anterior surface (102) including a spherical corrective portion (122) and an outer beveled portion (124), a posterior surface (103), and a flat outer edge surface (104) abutting the posterior surface and the beveled portion, wherein the flat outer edge surface is oriented in only the Z direction, parallel to the central axis.

2. The corneal inlay of claim 1, wherein the posterior surface (103) is spherical.

3. The corneal inlay of claim 1, wherein the central thickness is about 30 microns.

4. The corneal inlay of claim 1, wherein the posterior surface (103) has a posterior radius of curvature of about 7.5 mm.

## Patentansprüche

1. Hornhaut-Inlay für die Behandlung von Presbyopie, umfassend:
einen Durchmesser (114) von 1 mm bis 5 mm;
einen Brechungsindex zwischen etwa 1,36 und 1,39; und
eine vordere Fläche (102) umfassend einen sphärischen Korrekturbereich (122) und einen äußeren abgeschrägten Bereich (124), eine hintere Fläche (103), und eine flache, an die hintere Fläche und den abgeschrägten Bereich angrenzende Außengrenzfläche (104), wobei die flache Außengrenzfläche nur in Z-Richtung orientiert ist, parallel zur Mittelachse.

2. Hornhaut-Inlay nach Anspruch 1, wobei die hintere Fläche (103) sphärisch ist.

3. Hornhaut-Inlay nach Anspruch 1, wobei die zentrale Dicke etwa 30 Mikron ist.

4. Hornhaut-Inlay nach Anspruch 1, wobei die hintere Fläche (103) einen hinteren Krümmungsradius von etwa 7,5 mm hat.

## Revendications

1. Prothèse cornéenne pour traiter la presbytie, comprenant :
un diamètre (114) de 1 mm à 5 mm ;
un indice de réfraction compris entre environ 1,36 et 1,39 ; et
une surface antérieure (102) comprenant une partie corrective sphérique (122) et une partie extérieure biseautée (124), une surface postérieure (103) et une surface de bord extérieur plat (104) qui vient en appui sur la surface postérieure et la partie biseautée, dans laquelle la surface de bord extérieur plat n'est orientée que dans la direction Z, parallèlement à l'axe central.

2. Prothèse cornéenne selon la revendication 1, dans laquelle la surface postérieure (103) est sphérique.

3. Prothèse cornéenne selon la revendication 1, dans laquelle l'épaisseur centrale est d'environ 30 micromètres.

4. Prothèse cornéenne selon la revendication 1, dans laquelle la surface postérieure (103) a un rayon de courbure postérieur égal à environ 7,5 mm.
